# EUROPEAN PATENT APPLICATION

(11) **EP 2 272 563 A1**
(43) Date of publication of application: **12.01.2011**
(21) Application number: 09164814.7
(22) Date of filing: 07.07.2009
(51) Int. Cl.: A61N 1/39, A61N 1/08

(54) **Protective defibrillator casing**

(71) Applicant: Pape, Gerson, 7586BW Overdinkel (NL)
(72) Inventor: Pape, Gerson, 7586BW Overdinkel (NL)
(74) Representative: de Lange, Hendrik Cornelis

(57) **Abstract**

The invention relates to a protective defibrillator casing, comprising a cover, a support structure suitable for supporting the defibrillator and/or the cover, a lock, suitable for locking the cover to the support structure, a receiver, suitable for receiving a signal from a network, a transmitter or a central operator, a processor in communicative connection with the receiver, configured to recognize said signal and configured to decide to disengage the lock based on predetermined criteria.

## Description

The invention relates to a casing of a defibrillator. More specific, the invention relates to a protective, fool proof protection of a defibrillator, which can be opened on demand and/or authorisation.

Defibrillation devices are for instance described in the European patent EP0569609. This document discloses a defibrillator having a power storage, such as a battery, two human contact electrodes, a load collector such as a capacitor and a switch, connecting the load collector with the human contact electrodes, for instantaneous load delivery to the human body, in order to revive e.g. a heart arrest patient.

More and more, the awareness of lethal heart arrest and the relative easy cure by defibrillation, urges governments and institutions to provide an increasing number of defibrillation stations with mobile defibrillators such as described in the above cited European patent.

However these defibrillator stations are prone to theft and vandalism.

Accordingly it is an object of the invention to mitigate or solve the above described and/or other problems of mobile defibrillator stations in the art, while maintaining and/or improving the advantages thereof.

More specifically it is an object to safely provide heart arrest rescue devices, while reducing the possibility of uncontrolled theft or vandalism.

This object is reached by a protective defibrillator casing, comprising a casing, a support structure suitable for supporting the defibrillator and/or the casing, a cover, a lock, suitable for locking the cover to the support structure, a receiver, suitable for receiving a signal from a network, a transmitter or a central operator and a processor in communicative connection with the receiver, configured to recognize said signal and configured to decide to disengage the lock based on predetermined criteria.

Thus the defibrillator is protected by a casing that can be opened after the processor receives a signal to do so. By this feature unauthorised opening is prevented, such that vandalism and theft of this indispensible life saving equipment can be reduced.

A further aspect of the invention is that the lock comprises an electro mechanical opener. Thus the lock can be opened by a signal given by the processor and can more practically be reclosed.

In another aspect of the invention, the processor is configured to recognize and/or to identify the origin of the signal. Thus an authorisation of a central operator centre or a telecommunication centre can be required. If the origin of the signal can be recognised, the user can be identified as well. After inspection of the casing and the defibrillator, eventual vandalism or theft might be traced back to the person culpable.

In a further aspect of the invention, the receiver is configured to receive signals originating from a mobile phone, a PDA or another mobile device, that is suitable for identification of the owner or the user of the device. By these specific features, a user can call a number that is for instance indicated on the cover of the casing, and after calling this number, being authorised to have the cover of the casing opened.

Yet another aspect of the invention is that the casing is provided with a visible and/or tactile code. This code can be requested to be typed in or called, in order to gain connection with an operator of a service maintaining the defibrillators. This code can comprise a telephone number, an SMS-number or other dialling code.

In the casing, the cover can be connected to the support structure by means of a hinge and/or a sliding cylinder.

A further aspect of the invention is that the processor is configured to generate a signal, which can comprise information such as e.g. a battery loading level, a logging on usage of the defibrillator, an alarm on theft or vandalism of the defibrillator and/or the casing. Thus a control and check system or an operator can remotely inspect the conditions of the defibrillator in question.

Yet another aspect of the invention is that a key switch is provided in the casing, wherein the key switch is configured to receive a key, wherein the key switch is further configured to overrule the processor and to unlock the lock when the key is inserted in the key switch and e.g. turned. Thus a maintenance person can be equipped with a master key, enabling him to open all casings in, for instance, a dedicated area.

In order to further elucidate the invention, exemplary embodiments will be described with reference to the drawing. In the drawing:
Figure 1 represents a schematic side view of a protective casing according to a first embodiment of the invention;
Figure 2 represents a schematic perspective view of a protective casing in an opened position according to a first embodiment of the invention;
Figure 3 represents a schematic front view of a protective casing according to a first embodiment of the invention;
Figure 4 represents a schematic perspective view of a protective casing according to a first embodiment of the invention;
Figure 5 represents a schematic perspective worked open view of a part of a protective casing according to a further embodiment of the invention;
Figure 6 represents a schematic perspective worked open view of a part of a protective casing according to a further embodiment of the invention, with a lock in a locking position;
Figure 7 represents a schematic perspective worked open view of a part of a protective casing according to a further embodiment of the invention, with a lock in a opening position.

The figures represent specific exemplary embodiments of the inventions and should not be considered limiting the invention in any way or form. Throughout the figures the same or corresponding reference numerals are used for the same or corresponding elements.

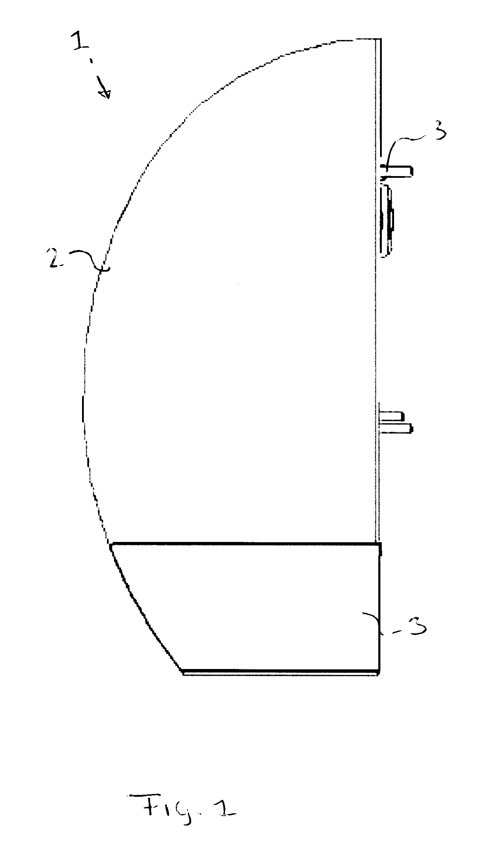

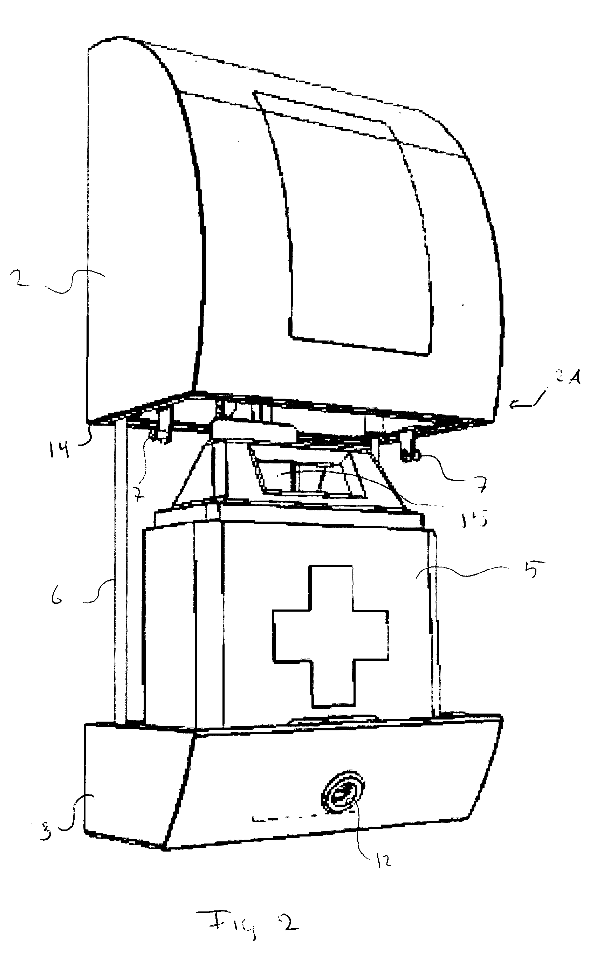

In figure 1, a side view of a casing 1 is presented. The casing 1 comprises a cover 2 and a support structure 3. The cover 2 can close off the casing by for example a rubber strip 14 provided at the lower edge of the cover 2 or the upper edge of the cup shaped portion 3a of the support structure 3. In a closed position, the cover thus seals off the support structure and protects the defibrillator 5 (see figure 2) from e.g. dust and/or moisture ingress.

In figure 2, the casing as represented in figure 1 is presented in an opened position. The cover 2 can slide along sliding cylinders 6 over the contents of the casing and making its contents accessible. The sliding cylinders can be for instance gas pressure cylinders, which can be e.g. gas pressure loaded in a closed position of the cover 2, thus enabling a quick and practical opening of the cover 2. The rapid and uncomplicated opening of the cover 2, is a valuable feature, since persons using the mobile defibrillator 5 are under high stress levels and under time pressure rescuing a person suffering a heart arrest. For these reasons, it is helpful when the cover rapidly and uncomplicatedly opens.

The cylinders 6 can alternatively also be spring loaded or provided with mild explosive charges in order to provide a rapid deployment.

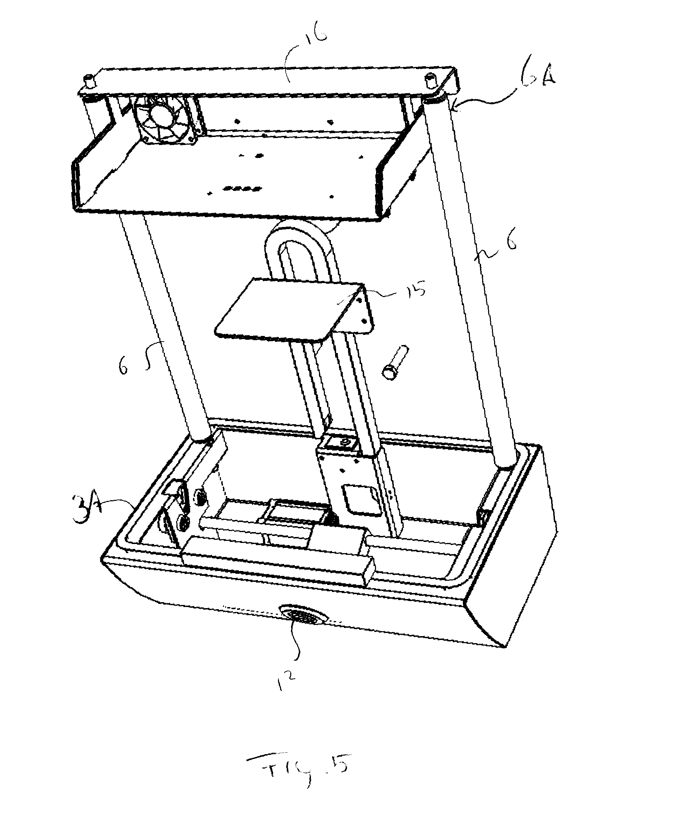

Within the casing the mobile defibrillator 5 can be hooked up to a bracket 15 (see figure 5) in order to remain it position within the casing 1.

Figure 2 depicts the defibrillator casing 1 in an open position. At the closing portion 2a of the cover 2, two locking pins 7 can be provided which can be engaged in the locks 8. In the support structure, a key switch 12 can be provided. In this key switch 12, a master key can fit, enabling the proprietor of the master key to open all protective defibrillator cases he/she is responsible for.

Although the key switch 12 is provided at the front end of the support structure 3 of the protective casing 1, it can similarly be arranged in another part of the cover 2 or the support structure 3.

The casing 1 is specially configured such that regular mobile defibrillators 5 will fit in easily. If defibrillators 5 of different manufactures are used, the bracket 15 may be adapted or exchanged in order to provide a dedicated defibrillator positioning element, providing optimal fixation of the defibrillator when the protective casing 2 is closed, while providing optimal disengagement of the defibrillator 5 when the casing 2 is opened.

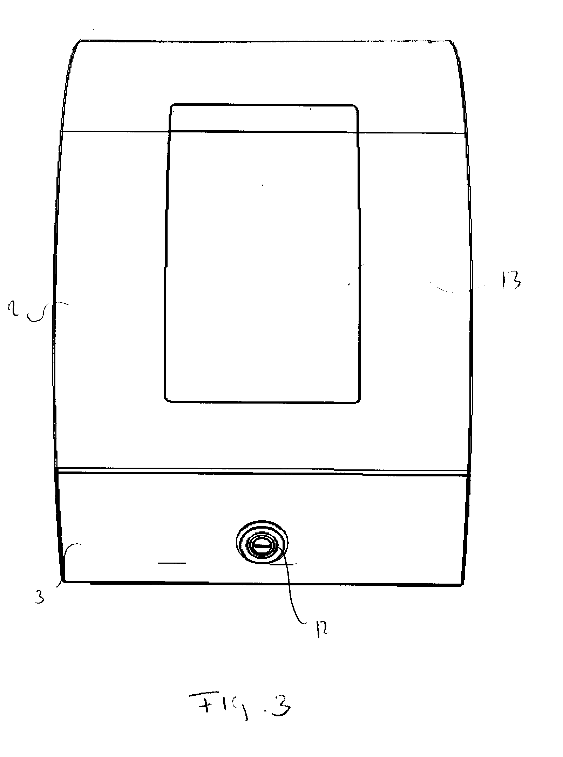

In figure 3 a front side view of the protective defibrillator casing 1 is provided. On the cover 2 an instruction area 13 can be provided, which can for example provide in a pictogram style, the functioning of the casing 1, how to open it and how to deploy and operate the defibrillator contained therein. The area 13 can additionally or alternatively be provided with emergency telephone numbers, unlocking instructions, unlocking codes, and/or unlocking dialling codes.

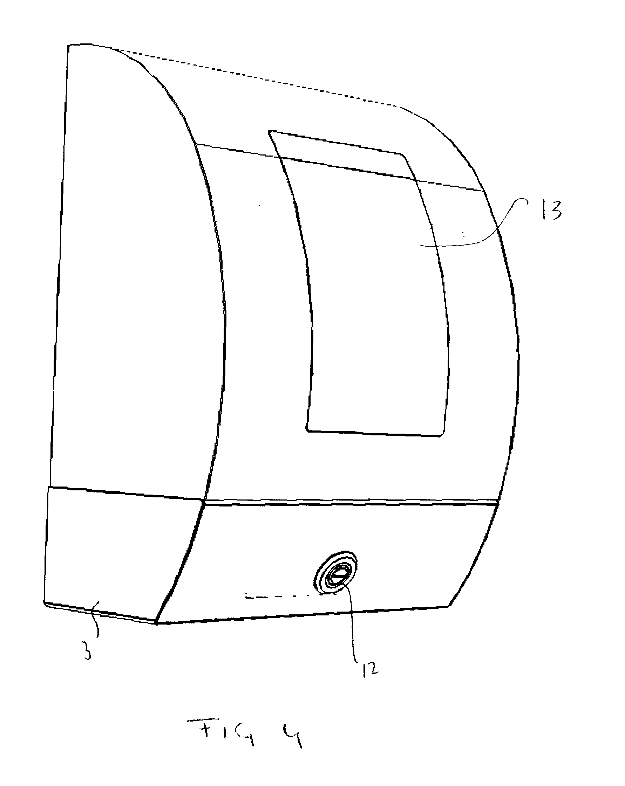

In figure 4 a perspective view of the protective casing 2 is presented. The shape of the cover is chosen to be provided with rounded off corners. These rounded off corners provides less hold to potential vandals or crooks trying to remove the complete casing 1 and can help prevent e.g. rain or moisture ingress. Furthermore the shape of the curvature of the cover 2 can be chosen so that no snow or ice can collect there upon.

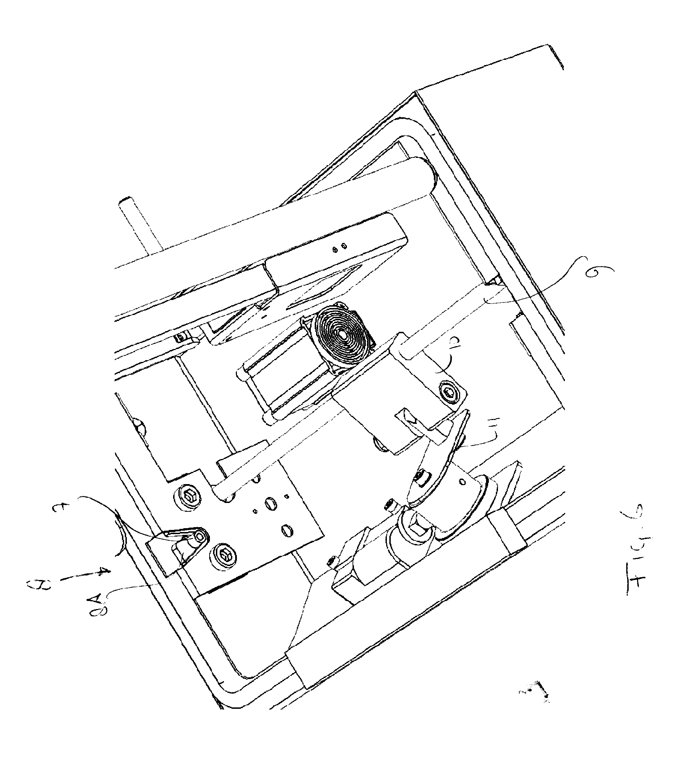

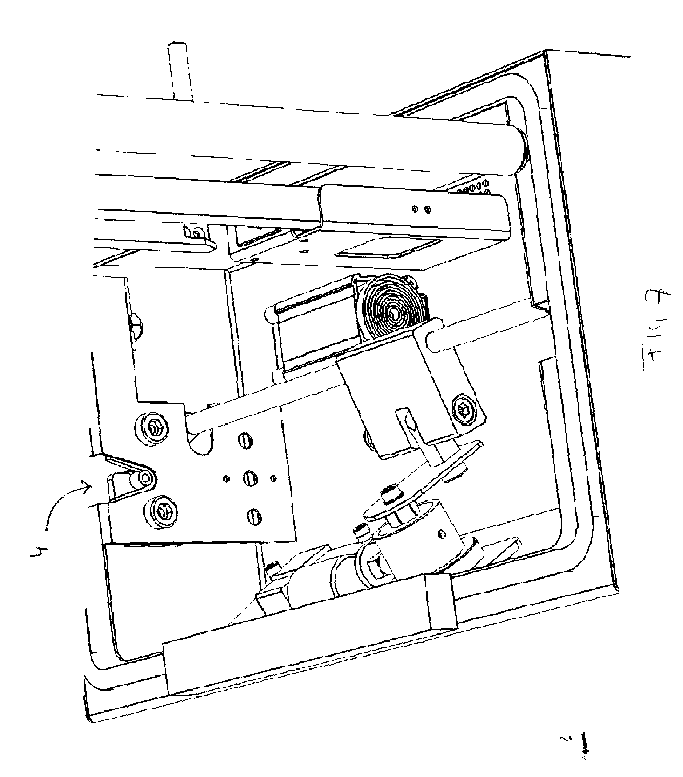

In figures 5-7 detailed views of the support structure 3 are depicted. On this structure, two guiding cylinders 6 are mounted. On the opposite distal end 6a of the guiding cylinders 6, a bracket 16 is mounted for fixing the cover 2. In the lower cup shaped portion 3a of the support structure 3, two locks 8 are provided. These locks 8 can be opened by means of a push bar 9, which can be controlled with an electromagnetic opener 11, which can operate the push bar 9 through connector 10.

The engaging clamps 8a can be connected to the push bar 9 such that the engaging clamps 8a move aside, disengaging the locking pins 7 within the lock 8, such that the cover 2 is no longer fixed on the support structure 3. By disengaging the locking pins 7, the pressure or spring loaded cylinders 6 can relax and push the cover 2 upward.

The invention is to be understood not to be limited to the exemplary embodiments shown in the figures and described in the specification. For instance the casing 1 is disclosed to be applied for mobile defibrillators 5, however also other goods can be protected therein. For instance post, packets, valuables, gemstones, jewels, money, keys etc.

Thus in an alternative arrangement, a multitude of boxed can be presented, each with an own dedicated code. These boxes can for instance be booked, opened and closed by a mobile communicator, such as a mobile phone or PDA.

Eventually besides the booking, opening and closing, even the billing for the time the container is rented out can be performed through the operators of a mobile network, in which the mobile communicator is active.

These and other modifications are considered to be variations that are part of the framework, the spirit and the scope of the invention outlined in the claims.

## Claims

1. A protective defibrillator casing (1), comprising
- a cover (2);
- a support structure (3) suitable for supporting the defibrillator (5) and/or the cover (2);
- a lock (8), suitable for locking the cover (2) to the support structure (3);
- a receiver, suitable for receiving a signal from a network, a transmitter or a central operator;
- a processor in communicative connection with the receiver, configured to recognize said signal and configured to decide to disengage the lock (8) based on predetermined criteria.

2. A protective defibrillator casing (1) according to claim 1, wherein the lock (8) comprises an electro mechanical opener (11).

3. A protective defibrillator casing (1) according to any of the preceding claims, wherein the processor is configured to recognize and/or to identify the origin of the signal.

4. A protective defibrillator casing according to claim 2 or 3, wherein the processor is in operational connection with the electro mechanical opener (11).

5. A protective defibrillator casing (1) according to any of the preceding claims, wherein the receiver is configured to receive signals originating from a mobile phone, a PDA or another mobile device, that is suitable for identification of the owner or the user of the device.

6. A protective defibrillator casing (1) according to any of the preceding claims, wherein the casing (1) is provided with a visible and/or tactile code.

7. A protective defibrillator casing (1) according to claim 6, wherein the code comprises a telephone number, an SMS-number or other dialling code.

8. A protective defibrillator casing (1) according to any of the preceding claims, wherein the cover (2) is connected to the support structure (3) by means of a hinge and/or sliding cylinders (6).

9. A protective defibrillator casing (1) according to any of the preceding claims, wherein the processor is configured to generate a signal.

10. A protective defibrillator casing (1) according to claim 9, wherein the signal comprising information such as e.g. a battery loading level, a logging on usage of the defibrillator (5), an alarm on theft or vandalism of the defibrillator (5) and/or the casing (1).

11. A protective defibrillator casing (1) according to claim 6, wherein a key switch (12) is provided configured to receive a key, wherein the key switch (12) is further configured to overrule the processor and to unlock the lock (8) when the key is inserted in the key switch and e.g. turned.
